**Europäisches Patentamt**

**European Patent Office**

**Office européen des brevets**

(19)

(11) Veröffentlichungsnummer: **0 200 069 B1**

(12) **EUROPÄISCHE PATENTSCHRIFT**

(45) Veröffentlichungstag der Patentschrift: **10.07.91**

(51) Int. Cl.⁵: **C12P 19/24**, C12N 9/90

(21) Anmeldenummer: **86105091.2**

(22) Anmeldetag: **14.04.86**

(54) **Kontinuierliches Verfahren zur enzymatischen Herstellung von Isomaltulose.**

(30) Priorität: **27.04.85 DE 3515284**
**10.08.85 DE 3528752**

(43) Veröffentlichungstag der Anmeldung:
**10.12.86 Patentblatt 86/45**

(45) Bekanntmachung des Hinweises auf die
Patenterteilung:
**10.07.91 Patentblatt 91/28**

(84) Benannte Vertragsstaaten:
**AT BE CH DE FR GB IT LI NL SE**

(56) Entgegenhaltungen:
**EP-A- 0 001 099**
**EP-A- 0 028 900**
**EP-A- 0 049 801**
**EP-A- 1 022 07**
**FR-A- 2 179 966**

**TECHNISCHE RUNDSCHAU, Band 77, Nr. 38,**
**17. September 1985, Seiten 38,39,42,43, Bern,**
**CH; U. BEERSTECHER: "Forschung und Inno-**
**vation auf der Achema 1985"**

(73) Patentinhaber: **SÜDZUCKER AKTIENGESELL-**
**SCHAFT MANNHEIM/OCHSENFURT**
**Maximilianstrasse 10**
**W-6800 Mannheim 1(DE)**

(72) Erfinder: **Egerer, Peter, Dr.**
**Ilching 15**
**W-8011 Kirchseeon(DE)**
Erfinder: **Crueger, Wulf, Dr.**
**Ruhrstrasse 35**
**W-4006 Erkrath 2(DE)**
Erfinder: **Schmidt-Kastner, Günter, Prof. Dr.**
**Falkenberg 59**
**W-5600 Wuppertal 1(DE)**

(74) Vertreter: **Körber, Wolfhart, Dr. et al**
**Patentanwälte Mitscherlich & Partner Steins-**
**dorfstrasse 10**
**W-8000 München 22(DE)**

## Beschreibung

Gefunden wurde ein kontinuierliches Verfahren zur enzymatischen Herstellung von Isomaltulose in einem steril geführten Reaktorsystem, welches dadurch gekennzeichnet ist, daß man in einem steril führbaren Reaktorsystem

a) durch Fermentation Saccharose-Mutase-bildender Mikroorganismen Saccharose-Mutase herstellt, wobei die Zellen bereits vor Ende der logarithmischen Wachstumsphase mit Salzlösung und oberflächenaktiven Reagentien behandelt werden,

b) in an sich bekannter Weise durch Zellaufschluß und Cross-Flow-Mikrofiltration einen zellfreien Enzymrohextrakt herstellt,

c) die Saccharose-Mutase aus diesem Rohextrakt durch selektive Bindung an eine anionisierbare Trägermatrix gleichzeitig reinigt und immobilisiert, und

d) die dabei erhaltene Matrix mit Saccharose in Berührung bringt.

Nach der DE-PS 1 049 800 wird Saccharose durch Enzyme mikrobiellen Ursprungs in Isomaltulose umgewandelt. Neben Protaminobacter rubrum sind weitere Bakterien wie Erwinia carotovora, Serratia marcescens, Serratia plymuthica und Leuconostoc mesenteroides zu dieser Umlagerung befähigt [ S. Schmidt-Berg-Lorenz, W. Mauch, Z. Zuckerindustrie 14, 625-627 (1964): F.H. Stodola, 126th Meeting Amer. Chem. Soc. Sept. 1954, Abstr. of Papers S. D 5: W. Mauch, S. Schmidt-Berg-Lorenz, Z. Zuckerindustrie 14, 309-315 sowie 375-383 (1964)].

Darauf aufbauend wurden verschiedene Verfahren zur mikrobiellen oder enzymatischen Herstellung von Isomaltulose bekannt.

In der EP-PS 1099 wird zum Beispiel ein Verfahren zur kontinuierlichen Fermentation von Mikroorganismen mit gleichzeitiger Umsetzung von Saccharose zu Isomaltulose beschrieben. Die US-PS 4 386 158, DE-OS 3 038 219, EP-PS 28 900 beschreiben verschiedene Verfahren zur Herstellung von Isomaltulose mit immobilisierten Mikroorganismen.

In der DE-OS 3 038 218 wird erstmals das Enzym Saccharose-Mutase beschrieben und charakterisiert sowie dessen Immobilisierung und Verwendung zur Herstellung von Isomaltulose aufgezeigt.

Die Isolierung des Enzyms Saccharose Mutase wird dabei diskontinuierlich in einem mehrstufigen Verfahren und die nachfolgende Immobilisierung in einem separaten Reaktionsschritt beschrieben. Insbesondere die dabei verwendete Chromatographie an CM-Sephadex C-50 ist zeitaufwendig. Der Fermentationsschritt, der Zellaufschluß, die Enzymreinigung und die Immobilisierung des gereinigten Enzyms erfolgen dann in einer langwierigen Serie von Verfahrensschritten, die zudem nur schwierig unter keimfreien Bedingungen durchführbar sind.

Bei den bisher beschriebenen Methoden handelt es sich um insterile Verfahren zur Herstellung von Isomaltulose durch immobilisierte Mikroorganismen oder durch immobilisierte Saccharose-Mutase, welche zudem nur diskontinuierlich von der Fermentation bis zum Endprodukt Isomaltulose geführt wurden.

Bei dem erfindungsgemäßen Verfahren handelt es sich dagegen um ein kontinuierliches Vefahren in einem steril geführten Reaktorsystem zur enzymatischen Herstellung von Isomaltulose,

bestehend aus der Produktion periplasmatischer Saccharose-Mutase durch Fermentation Saccharose-Mutase-bildender Mikroorganismen,

der Herstellung des zellfreien Enzymrohextrakts durch Aufschluß der Zellen und durch Cross-Flow Mikrofiltration,

der einstufigen, gleichzeitigen Reinigung und Immobilisierung der Saccharose-Mutase aus dem durch Diafiltration konditionierten zellfreien Rohextrakt durch selektive Bindung an eine anionisierbare Trägermatrix,

und der direkten Umsetzung von Saccharose zu Isomaltulose durch die an entsprechende anionisierbare Trägermatrix, bevorzugt in Patronen- bzw. Kartuschenform, gebundene Saccharose-Mutase.

Insbesondere die Verwendung eines sulfonsauren Kationenaustauschers in Form einer Gewebematrix ermöglicht die außerordentlich selektive Bindung der Saccharose-Mutase aus dem Enzymrohextrakt bei höchsten Durchflußraten und führt dadurch zur Enzymreinigung in einem Schritt. Zur Verwendung der Saccharose-Mutase als Biokatalysator zur Umsetzung von Saccharose zu Isomaltulose braucht das Enzym nicht mehr vom Kationenaustauscher eluiert und konzentriert zu werden. Die anionisierbare Trägermatrix, insbesondere ein sulfonsaurer Kationenaustauscher, bindet die Saccharose-Mutase so fest, daß ein Betrieb auch als Bioreaktor über längere Zeit ohne Aktivitätsverlust möglich ist (Beispiel 1).

Besonders vorteilhaft ist die Verwendung einer sulfonsauren Matrix in Patronen- oder Kartuschenform, wie sie z.B. von der AMF, Molecular Separations Division, Meriden, CT, USA, in den verschiedenen handelsüblichen Formen ZetaPrep 100-SP® oder ZetaPrep 3000-SP® angeboten wird. Dabei handelt es sich um quervernetzte, sulfonsaure Cellulose, die zu Papierfolien verarbeitet und aufgewickelt in Patronenform vorliegt.

In ähnlicher Weise lassen sich anionische Sterilfilterpatronen verschiedener Hersteller, z.B. das Ultipor GF® Filtermedium der Fa. Pall verwenden. Hierbei ist ein Glasfasermaterial von einem Harz mit stark negativ geladenen funktionellen Gruppen umgeben.

Im Vergleich zur AMF ZetaPrep-SP® Systemserie, die zu chromatographischen Zwecken auf den Markt gebracht wurde, handelt es sich jedoch bei den handelsüblichen Sterilfilterpatronen um gefaltete Membranen oder Gewebe, wobei der Substratstrom nur eine Membran- oder Gewebeschicht passiert. Bei einer zylindrisch aufgewickelten Gewebe-oder Membranfolie, dem Charakteristikum einer bevorzugten Variante des erfindungsgemäßen Verfahren, muß der Substratstrom hingegen viele Gewebe- oder Membranschichten radial zur Zylinderachse hin passieren.

Die bei der selektiven Isolierung der Saccharose-Mutase aus dem Rohextrakt möglichen hohen Flußraten sind ebenfalls vorteilhaft für die anschließende Reaktorführung mit hochprozentiger Saccharoselösung. Dies ist eine der Voraussetzungen für eine hohe Produktivität des Saccharose-Mutase-Bioreaktors.

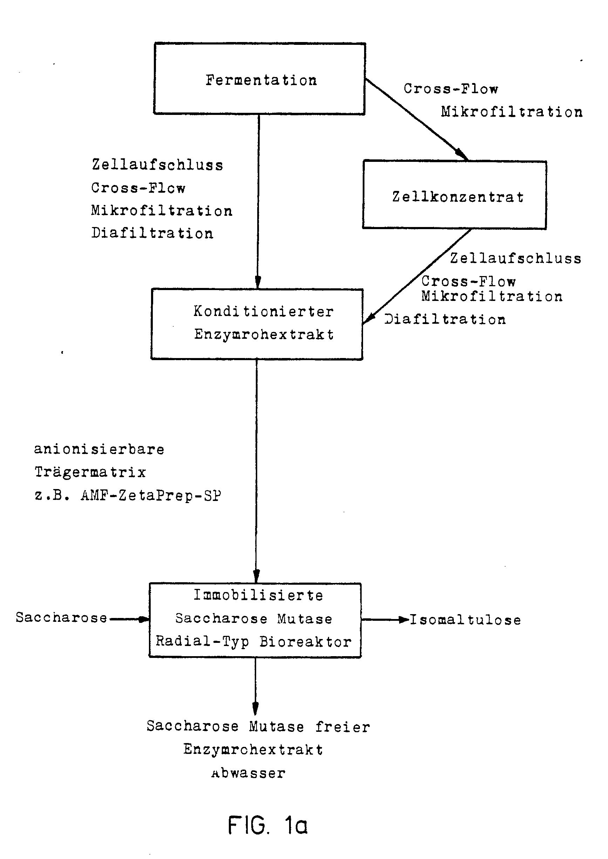

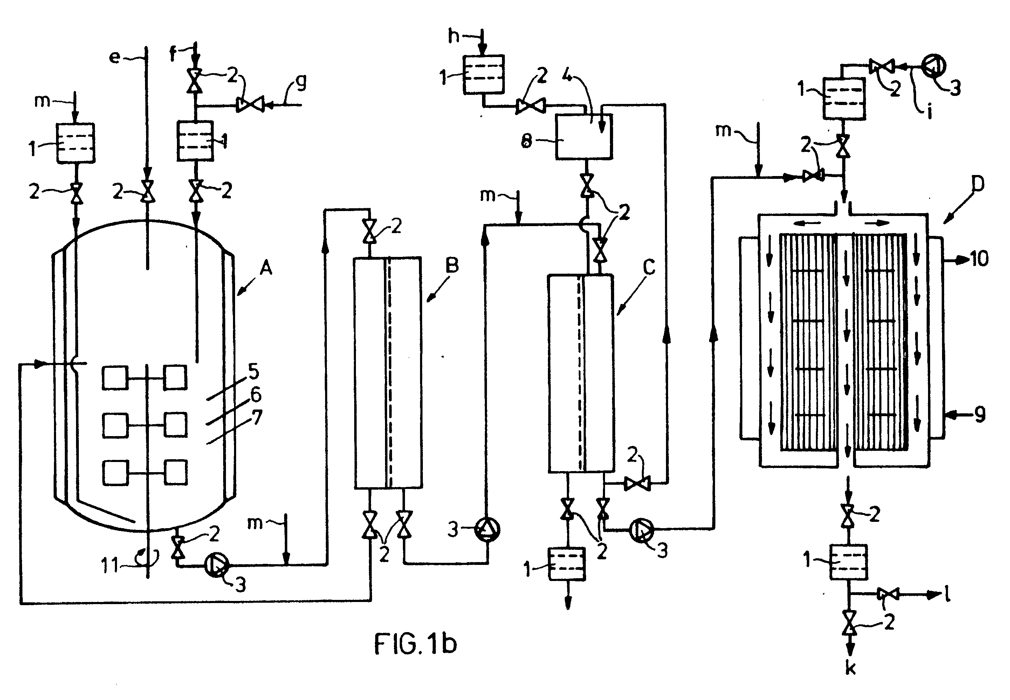

Die Verbindung des Fermentationsschrittes, des Zellaufschlusses, des einstufigen, gleichzeitigen Aufarbeitungsund Immobilisierungsverfahrens und des Biotransformationsschrittes ermöglicht dementsprechend ein kontinuierliches Gesamtverfahren zur enzymatischen Herstellung von Isomaltulose, welches auf sehr einfache Weise keimfrei zu betreiben ist (Fig. 1 a,b).

Ein steril geführtes Gesamtverfahren zur enzymatischen Herstellung von Isomaltulose, welches besonders vorteilhaft durch die erfindungsgemäßen Verfahrensschritte verwirklicht wird, verhindert den Eintritt von Keimen in den Bioreaktor und damit die Bildung von Nebenprodukten aus dem Substrat Saccharose. Meist handelt es sich bei derartigen Nebenprodukten um organische Säuren, mikrobielle Stoffwechselprodukte, die zur Absenkung des pH-Wertes im Bioreaktor und damit zur Inaktivierung der Saccharose-Mutase führen können.

Die Saccharose-Mutase verliert bei Temperaturen größer +40°C in kürzester Zeit ihre katalytische Aktivität, so daß Autoklavierung als Entkeimungsmethode ausscheidet. Die Verwendung der meisten Bakterizide oder Fungizide zur Keimabtötung würde wegen der Verwendung des Produktes Isomaltulose als Zuckeraustauschstoff eine sehr aufwendige Produktreinigung und diesbezügliche Analytik erfordern.

Besonders vorteilhaft ist es, wenn die Kationenaustauscher-Matrix eine hohe Konzentration an sulfonsauren funktionellen Gruppen aufweist. Gemäß Beispiel 1 konnten an eine AMF ZetaPrep 100-SP® Einheit mehr als 1 g reine Saccharose-Mutase gebunden werden. Laut Spezifikation des Herstellers beträgt beispielsweise die Kapazität der ZetaPrep 100-DEAE® Einheit 4-6 g Rinderserum Albumin [AMF Inc., LC.10.10, 30.11.1984]. Die obere Grenze der Kapazität dürfte demzufolge für Saccharose-Mutase ebenfalls bei etwa 4 g pro ZetaPrep 100-SP® liegen.

Für das Fabrikat AMF ZetaPrep ® werden etwa 30 Reaktorvolumina/h als maximale Durchflußraten für die Chromatographie proteinhaltiger wäßriger Lösungen angegeben [AMF Inc., LC.10.1G, 28.9.1983].

Gemäß Beispiel 1 wurden 7 Reaktorvolumina pro Stunde bei Durchsatz einer 50% Saccharoselösung mit Hilfe einer laborüblichen peristaltischen Pumpe erzielt. Bei Verwendung leistungsfähigerer Pumpen, z.B. der in der Zuckerindustrie häufig verwendeten Zahnradpumpen, ist eine Mehrförderung zur Ausnützung höherer Enzymbeladungen möglich.

Allgemein ist für eine katalytische Reaktion von erheblicher Bedeutung, das Reaktorvolumen so klein wie möglich zu halten, so z.B. das Festbettvolumen eines immobilisierten Biokatalysators in Partikelform. Das erfordert einen Biokatalysator hoher Aktivität pro Einheitsvolumen bzw. hoher Beladungsdichte oder Aktivitätsdichte.

Dadurch ist es möglich, in kleineren Reaktoreinheiten zu produzieren und damit erhebliche Anlagen-, Energie- und Wartungskosten zu sparen.

Auf Grund des erfindungsgemäßen Verfahrens lassen sich Produktströme von 5-10 Reaktorvolumina/h für die Umwandlung von Saccharose zu Isomaltulose bei Einsatz 50%-iger Saccharoselösung erzielen (Beispiel 1). Vergleichbar dazu liegen mit immobilisierten Zellen mögliche Produktströme in der Größenordnung von 0.5-1.0 Reaktorvolumina/h.

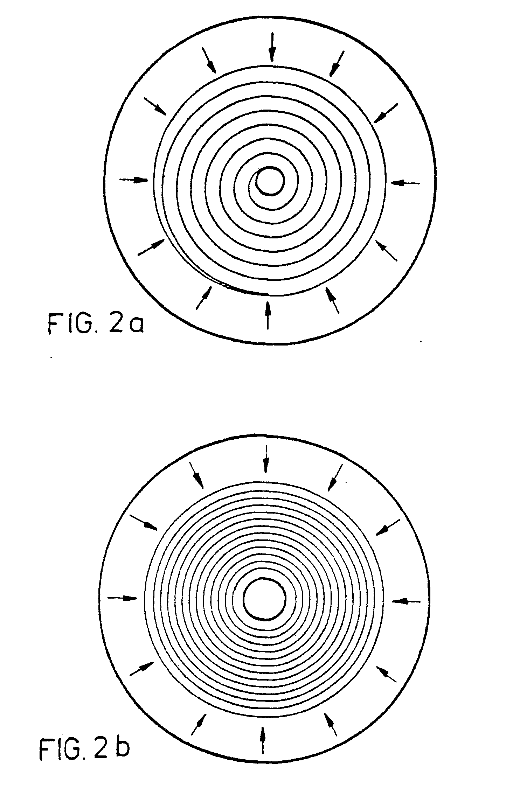

Das erfindungsgemäße Verfahren beinhaltet deshalb auch die Verwendung eines Radial-Typ-Bioreaktors für Biotransformationen. Wie Fig. 2 a und b zeigt, wird der Substratstrom durch die verschiedenen Wicklungen des Cellulosegewebes im zylindrischen Reaktorgefäß radial zur Zylinderachse hin dem Auslauf zugeführt, wobei beim Durchtritt durch das Cellulosegewebe ein Flächengradient pro Wicklung und damit ein Verweilzeitgradient über die gesamte Gewebefläche vorhanden ist.

Die Durchtrittsgeschwindigkeit durch das Cellulosegewebe nimmt zu von der äußersten zur innersten Wicklung hin; die Gewebefläche pro Wicklung nimmt dabei ab. Bei gleichmäßiger Enzymbeladung bedeutet dies eine abnehmende Enzymmenge pro Wicklung zur innersten Wicklung hin. Die Verweilzeit des Massenstroms ist daher bei hohem Umsatz, also bei hoher Produktkonzentration und geringer Substratkon-

3

zentration, geringer als bei niedrigem Umsatz, also bei geringer Produktkonzentration und hoher Substratkonzentration. Dies reduziert die enzymatisch bedingte Nebenproduktbildung, wie sie bei der Saccharose-Mutase-katalysierten Umlagerung von Saccharose zu Isomaltulose anhand der gebildeten Glucose, Fructose, 1,1'-Disaccharid, Oligosaccharide beobachtet wird.

Wie aus Beispiel 1 ersichtlich, ist bei vollständigem Saccharoseumsatz die Konzentration an Glucose (etwa 1,0 %) und Fructose (etwa 2,0 %) geringer als bei Vergleichsansätzen mit anderweitig immobilisierter Saccharose-Mutase [vergleiche DE-OS 3 038 218], worin ein weiterer Vorteil des erfindungsgemäßen Verfahrens liegt.

Das erfindungsgemäße Verfahren ist insbesondere auch dadurch gekennzeichnet, daß zur Gewinnung des Enzyms aus den Bakterienzellen (Herstellung des Enzymrohextrakts) die Behandlung mit NaCl, KCl oder anderen Salzen allein oder in Kombination mit oberflächenaktiven Verbindungen wie Tween, Span, Brij ausreicht.

Für die Ausbeute bei der Herstellung des Enzymrohextrakts ist jedoch das Alter der Fermentation entscheidend.

Wie Tabelle 1 zeigt, nimmt das Verhältnis von Saccharose-Mutase im zellfreien Überstand nach Salz- und Tweenbehandlung zu Gesamtaktivität der Zellsuspension mit dem Alter der Zellkultur ab. Es besteht damit ein entscheidender Zusammenhang zwischen der Güte des Zellaufschlusses, der Enzymausbeute und dem Fermentationsverlauf.

## Tabelle 1:

### Zellaufschluß Protaminobacter rubrum

Ausbeute an Saccharose-Mutase im zellfreien Überstand (Enzymrohextrakt) nach Aufschluß mit Salzlösung und oberflächenaktiven Reagentien in Abhängigkeit von der Fermentationszeit.

Bezugsgröße: Volumenaktivität der Fermentationslösung (U/ml) = 100 %.

| Fermentations- zeit | Volumenaktivität der Fermentations- lösung | Saccharose-Mutase im zellfreien Über- stand nach Auf- schluß (%) |
|---|---|---|
| 2 h | 0.7 U/ml | 31 % |
| 4 h | 1.4 U/ml | 102 % |
| 6 h | 5.6 U/ml | 72 % |
| 8 h | 14.4 U/ml | 79 % |
| 10 h | 14.8 U/ml | 55 % |
| 12 h | 13.1 U/ml | 30 % |

Das erfindungsgemäße Verfahren ist insbesondere auch dadurch gekennzeichnet, daß die selektive Bindung der Saccharose-Mutase aus dem Rohextrakt im pH-Bereich 4-12, bevorzugt zwischen pH 5-9, bei Salzkonzentrationen geringer als 0.2 M, bevorzugt bei 1 $\mu$M-10mM erfolgt. Der Enzymrohextrakt wird deshalb zuvor zweckmäßigerweise durch Diafiltration auf die entsprechende Salzkonzentration gebracht und für die einstufige Reinigung und Biotransformation konditioniert. Die selektive Bindung an sulfonsaure Kationenaustauscher-Matrizen kann auch in Gegenwart einer hochprozentigen Saccharoselösung, z.B. 40-60 %, erfolgen.

Das erfindungsgemäße Verfahren beschränkt sich nicht auf Saccharose-Mutase, die an eine sulfonsaure Matrix rein ionisch oder adsorptiv gebunden ist. Vielmehr kann es vorteilhaft sein, die aus dem Enzymrohextrakt selektiv gebundene Saccharose-Mutase an der sulfonsauren Matrix durch Behandlung mit Vernetzungsmitteln, wie z.B. durch polyfunktionelle Aldehyde, z.B Glutaraldehyd, oder durch Hexamethylendiamin/Carbodiimid zu stabilisieren.

Das erfindungsgemäße Verfahren ist insbesondere auch dadurch gekennzeichnet, daß das für die Umsetzung benötigte Enzym Saccharose-Mutase von Mikroorganismen vor allem von Protaminobacter rubrum [DSM (Deutsche Sammlung von Mikroorganismen) 2414], Erwinia carotovora (ATTC 25206) und Serratia plymuthica (ATTC 15928) gebildet wird. Dabei werden nach Animpfen der Mikroorganismen (0,1-10,0 % Impfmaterial) auf eine Kohlenhydrat-, Stickstoff- und anorganische Salze enthaltenden Nährlösung und Bebrütung unter optimalen Bedingungen die Fermentation vor Ende der log-Phase abgebrochen. Die Nährlösungen bestehen z.B. aus Dünnsaft/Dicksaft- oder/und Dünnsaft/Kläre-Gemisch aus einer Zuckerfabrik mit einem Trockensubstanzgehalt von 1-25 %, vorzugsweise 2-7 %.

Es wurde weiterhin festgestellt, daß das Ausbeuteoptimum der Saccharose-Mutase dann erzielt wird, wenn die Zellen bereits vor Ende der logarithmischen Wachstumsphase mit Salzlösung und oberflächenaktiven Reagentien behandelt werden. Fermentationsansätze, die schon in die stationäre Phase übergegangen sind, oder gealterte Fermentationsbrühen zeigen weniger günstiges Aufschlußverhalten bezüglich der Saccharose-Mutase: das Verhältnis der Saccharose-Mutase-Aktivität im zellfreien Überstand nach Behandlung mit Salzlösung und oberflächenaktiven Reagentien zur Gesamtaktivität der nicht aufgeschlossenen Zellen nimmt drastisch ab (Tabelle 1).

Fig. 1 a,b zeigen Fließschemata des erfindungsgemäßen Gesamtverfahrens. Es ist möglich, die Fermentationslösung sowohl direkt aufzuschließen als auch zuerst durch Cross-Flow-Mikrofiltration, etwa 1:10, zu konzentrieren und den Zellaufschluß danach bei reduziertem Zellsuspensionsvolumen durchzuführen. Die Cross-Flow-Mikrofiltration kann beispielsweise unter Verwendung der Produktreihe der Fa. Membrana oder der Fa. Millipore durchgeführt werden. Die Diafiltration wurde im Labormaßstab mit Hilfe der Amicon-Ultrafiltrationseinheit, Membran PM 10000, oder im Pilotmaßstab mit dem DDS-System, Membranausschluß MW 6000, durchgeführt.

Die in Fig. 1b gezeigte Reaktoranordnung zur kontinuierlichen, enzymatischen Herstellung von Isomaltulose umfaßt die Fermentereinheit, die Cross-Flow-Mikrofiltrationseinheit, die Diafiltrationseinheit und den Bioreaktor, der zur gleichzeitigen, einstufigen Reinigung und Immobilisierung der Saccharose-Mutase sowie zur nachfolgenden Biotransformation von Saccharose zur Isomaltulose eingesetzt wird.

In Fig. 1b sind die Reaktoren nicht maßstabgetreu zueinander dargestellt. Die Volumina der verschiedenen Einheiten richten sich nach den Erfordernissen der Isomaltuloseproduktion: Durchsatz an Saccharose, Produktivität des Bioreaktors, geforderte Produktionsmenge an Isomaltulose.

Zusammenfassend weist das erfindungsgemäße kontinuierliche Verfahren zur enzymatischen Herstellung von Isomaltulose folgende Vorteile gegenüber bisher bekannt gewordenen Verfahren auf:

- kontinuierliche Verfahrensführung von der Fermentation bis zum Produkt Isomaltulose möglich:
- sterile Führung des Gesamtverfahrens wesentlich begünstigt;
- Simultanschritt der Reinigung und Immobilisierung von Saccharose-Mutase durch selektive Bindung an Carboxylat-, Sulfat-, Phosphat- oder andere anionische Trägermaterialien, besonders vorteilhaft an sulfonsaurer Matrix;
- anionische Austauschermatrix in Gewebeform, speziell sulfonsaures vernetztes Cellulosegewebe in Patronenform, gewickelt als Radial-Typ-Bioreaktor, ermöglicht hohe Durchflußraten;
- derartig gebundene Saccharose-Mutase ist direkt zur Biotransformation Saccharose → Isomaltulose einsetzbar;
- Radial-Typ-Reaktor bewirkt auf Grund des Enzymaktivitätsgradienten zum Reaktorausgang hin eine Reduzierung der Konzentration der Nebenkomponenten.

Beispiel 1

Von einer Abimpfung des Stammes Protaminobacter rubrum Z 12 [DSM (Deutsche Sammlung von Mikroorganismen) 2414] werden Zellen mit 10 ml einer sterilen Mischung von einem Teil Dicksaft (TS = 7 %) und zehn Teilen Leitungswasser plus 0.5 g/l $(NH_4)_2HPO_4$ abgeschwemmt. Diese Suspension dient als Impfgut für die Schüttelmaschinenvorkultur in 1 l Kolben mit 200 ml Nährlösung obiger Zusammensetzung (Sterilisation 20 min, bei 121 °C). Nach einer 7-stündigen Bebrütungszeit bei 30 °C werden mit 2 Kolben (0.4 l) 20 l Nährlösung obiger Zusammensetzung in 30 l-Kleinfermenter beimpft und bei 30 °C mit 20 l Luft pro Minute und einer Rührgeschwindigkeit von 350 Upm fermentiert. Kurz vor Ende der log-Phase nach 8 Stunden wird die Kultur abgekühlt und aufgeschlossen. Dazu werden zunächst die 20 l Fermentationsbrühe (14.4 U/ml Saccharose-Mutase Aktivität) mit Hilfe des Pellicon-Systems, Durapore Filter (0.5 µm) (Fa. Millipore) durch Cross-Flow-Mikrofiltration auf etwa 3 l Zellsuspension (93.7 U/ml ) konzentriert.

Zu den 3 l Zellsuspension werden 3 l Salzlösung 0.5 M NaCl, 0.05 M K-Phosphat, pH 7.0, 1 % Tween 20, auf 30 °C angewärmt durch einen Sterilfilter gegeben und die Gesamtsuspension im Fermenter für 16 h bei Raumtemperatur mit 30 Upm gerührt. Danach wird die Gesamtsuspension mit 24 l sterilfiltriertem, entionisiertem Wasser verdünnt und über das Pelliconsystem zur Abtrennung der Zellen und Zelltrümmer gepumpt. Das Filtrat (7.6 U/ml) wird danach über einen Vorratsbehälter auf ein weiteres Pelliconsystem,

EP 0 200 069 B1

Polysulfonmembran 10000, (Fa. Millipore) gepumpt und auf etwa 3 l konzentriert. Der Fermenter wird erneut mit sterilem entionisiertem Wasser auf 30 l aufgefüllt, mikrofiltriert und das Filtrat nach Zumischung zum Vorratsbehälter über das Pelliconsystem, Polysulfonmembran, erneut auf 3 l konzentriert. Über das Vorratsgefäß werden nun mit etwa 60 l 1 mM K-Phosphat, pH 7.0, diafiltriert und dabei auf 9 1 sogenanntem konditionierten Enzymrohextrakt verdünnt (17.6 U/ml, 1,9 mg/ml Protein).

0.5 l davon werden sofort auf eine AMF-100 SP Kartusche mit einer Flußrate von 2.0 1/h gepumpt. Im Eluat sind weniger als 0.5 U/ml Protein, jedoch noch 1.9 mg/ml Protein meßbar, d.h., die Saccharose-Mutase ist nahezu selektiv aus dem Enzymrohextrakt an das vernetzte, sulfonsaure Gewebe der AMF-Kartusche gebunden worden.

Durch die AMF-Kartusche wird nun sterilfiltrierte 50 % Saccharoselösung bei Raumtemperatur, Flußrate 65 ml/h, gepumpt. Im Eluat sind noch etwa 10-12 %. Saccharose (% Anteil am Gesamtzucker) nachweisbar, der Umsatz beträgt somit 88-90 %. Nach 3 Tagen kontinuierlichem Betrieb wird der Bioreaktor zunächst auf 25°C, danach auf 30°C kontinuierlich geführt. Bei 25°C bzw. 30°C steigt der Umsatz stark an, die Durchsatzrate der 50 % Saccharoselösung wird deshalb auf 20-140 ml/h gesteigert. Der Saccharose umsatz bewegt sich dabei zwischen 65 und 75 %.

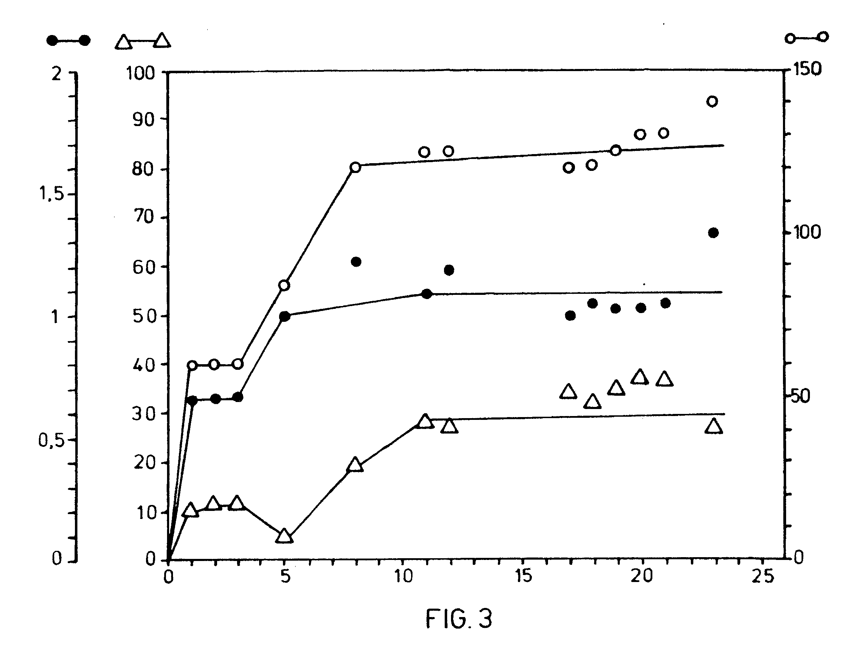

Dieses Langzeitexperiment ist in Fig. 3 dargestellt und zeigt, daß die Saccharose-Mutase in hochgereinigter Form unter diesen Betriebsbedingungen über etwa einen Monat unter Erhaltung ihrer Aktivität im oben beschriebenen Radial-Typ-Bioreaktor zur Biotransformation von Saccharose zu Isomaltulose eingesetzt werden kann.

Von dem im Vorratsgefäß gekühlt aufbewahrten Enzymrohextrakt (17.6 U/ml) werden nun weitere 2 l über obigen Bioreaktor gepumpt. Im Eluat waren lediglich 1.4 U/ml meßbar, somit etwa weitere 32000 U an der sulfonsauren AMF-Kartusche gebunden. Mit 50 %iger Saccharoselösung und bei einer Flußrate von etwa 210-220 ml/h ergab sich folgende Eluatzusammensetzung: 85,5 % Isomaltulose, 11,0 % 1,1'-Disaccharid, 2,5 % Fructose, 1,0 % Glucose; es war keine Saccharose nachweisbar.

Vom Vorratsgefäß wurden nun die restlichen 6,5 l Enzymrohextrakt über den Bioreaktor gepumpt. Etwa 1,2 U/ml wurden nicht gebunden, und der Bioreaktor somit mit weiteren 110000 U beladen.

Bei einer Durchflußrate von 610 ml/h wurde der Bioreaktor 5 Tage kontinuierlich betrieben und folgendes Produktspektrum gemessen: 85,2 % Isomaltulose, 10,2 % 1,1'-Di-saccharid, 2,1 % Fructose, 0,8 % Glucose.

Bei Erhöhung der Durchflußrate auf 700 ml/h ergaben sich 3,2 % Restsaccharose, 83,7 % Isomaltulose, 10,2 % 1,1'-Di-saccharid, 2,1 % Fructose und 0,6 % Glucose.

Bei etwa 97 % Umsatz bedeutet dies einen konstanten Durchsatz von etwa 7 Reaktorvolumina 50 % Saccharoselösung pro Stunde.

Zur Ermittlung der Beladungskapazität des Bioreaktors wurden erneut 20 l mit Protaminobacter rubrum beimpfte Kulturlösung fermentiert (15,6 U/ml), unter gleichen Bedingungen 9 l Enzymrohextrakt (22,1 U/ml) hergestellt und portionsweise über den Bioreaktor mit einer Flußrate von etwa 1,32 l/h geschickt.

Portion 1: 2 l, 1,9 U/ml im Eluat, somit 404000 U gebunden,

Portion 2: 2 l, 1,5 U/ml im Eluat, somit 41200 U gebunden,

Portion 3; 5 l, 1,5 U/ml im Eluat, somit 103000 U gebunden.

Wenn die in der DE-0S 3 038 218 angegebenen 450 U/mg elektrophoretisch nahezu reiner Saccharose-Mutase zugrundegelegt werden, bedeutet dies, daß etwa 335000 U etwa 0,75 9 reiner Saccharose-Mutase entsprechen und selektiv aus dem Enzymrohextrakt an die sulfonsaure AMF-Gewebematrix im 100 ml Bioreaktor gebunden werden konnte.

Beispiel 2

Von einer Abimpfung des Stammes Serratia plymuthica (ATCC 15928) werden Zellen mit 10 ml einer Dicksaftlösung (5 % TS-Gehalt, 0,5 g/l $(NH_4)_2HPO_4$-Zusatz) abgeschwemmt. Diese Suspension dient als Impfgut für eine Schüttelmaschinen-Vorkultur in einem 1 l-Kolben mit 200 ml steriler Nährlösung entsprechender Zusammensetzung. Nach 15-stündiger Bebrütungszeit bei 30°C werden mit je 10 Kolben (2 l) 18 l Nährlösung obiger Zusammensetzung in einem 30 l-Kleinfermenter beimpft und bei 30°C mit 20 l Luft/min. und einer Rührergeschwindigkeit von 350 Upm fermentiert. Kurz vor Ende der log-Phase nach 10 Stunden wird die Kultur abgekühlt.

Die Verfahrensschritte Zellaufschluß, Cross-Flow-Mikrofiltration, Diafiltration wurden analog Beispiel 1 durchgeführt. Lediglich die Konzentration an NaCl in der Salzlösung war 1 molar.

Daten einzelner Verfahrensschritte: 20 l Fermentationsbrühe 7,1 U/ml, 3 l Konzentrat 44,7 U/ml, 30 l Mikrofiltrat 3,9 U/ml, 10 l Retentat der Diafiltration 10,8 U/ml (Enzymrohextrakt).

Vom Retentat der Diafiltration (1 mM K-Phosphat, pH 7,0) wurden 1 l auf eine AMF-100 SP Kartusche,

EP 0 200 069 B1

Flußrate 1,2 l/h gepumpft. Im Eluat wurde eine Saccharose-Mutase-Aktivität von 0,45 U/ml gemessen. Bei kontinuierlichem Durchsatz von 50 % Saccharoselösung (sterilfiltriert) konnte bei einer Durchsatzrate von 90 ml/h bei 30°C ein Umsatz von 94,5 % Saccharose gemessen werden. Auch die weiteren 9 l des Enzymrohextraktes wurden über den Bioreaktor mit einer Flußrate von 1,2 l/h gepumpt. Das Eluat wies 0,4 U/ml Saccharose-Mutase auf.

Beispiel 3

Von einer Abimpfung des Stammes Erwinia carotovora (ATCC 25206) werden Zellen mit 10 ml einer Dicksaftlösung (5 % TS-Gehalt, 0,5 g/l $(NH_4)_2HPO_4$-Zusatz) abgeschwemmt. Diese Suspension dient als Impfgut für eine Schüttelmaschinen-Vorkultur in einem 1 l-Kolben mit 200 ml steriler Nährlösung entsprechender Zusammensetzung. Nach 30-stündiger Bebrütungszeit bei 30°C werden mit je 10 Kolben (2 l) 18 L Nährlösung obiger Zusammensetzung in einem 30-l-Kleinfermenter beimpft und bei 30°C mit 20 l Luft/min. und einer Rührergeschwindigkeit von 350 Upm fermentiert. Kurz vor Ende der log-Phase nach 18 h wird die Kultur abgekühlt und aufgeschlossen.

Das analog Beispiel 2 erhaltene Retentat der Diafiltration, ein Enzymrohextrakt der Aktivität 3,9 U/ml (Fermentationslösung 5,6 U/ml) im Gesamtvolumen 10 l, wurde über eine AMF-100 SP Kartusche bei einer Flußrate von 1,2 l/h gepumpt. Das Eluat wies 0,15 U/ml Saccharose-Mutase auf. Bei nachfolgendem kontinuierlichem Durchsatz von sterilfiltrierter 50 % Saccharoselösung, 190 ml/h, 30°C, wurden nur noch 2 % Restsaccharose (%-Anteil an Gesamtzucker) gemessen.

Legenden zu den Abbildungen

Fig. 1a:

Fließschemata des erfindungsgemäßen Verfahrens. Unter dem Begriff 'Konditionierter Enzymrohextrakt" sind zellfreier, Saccharose-Mutase-haltiger Kulturüberstand oder entsprechendes Diafiltrat zu verstehen, welche auf die zur selektiven Bindung an anionisierbare Trägermatrix nötigen Parameter, z.B. Salzkonzentration, Leitfähigkeit, pH-Wert, eingestellt wurden.

Der Begriff "Radial-Typ Bioreaktor" wird im Anmeldungstext in Verbindung mit Fig. a und b erläutert.

Fig. 1b:

Reaktoranlage zur kontinuierlichen, enzymatischen Herstellung von Isomaltulose (nicht maßstabgetreu).
Die Anlage besteht aus 4 Reaktoruntereinheiten.
(A) dem Fermenter
(B) der Anlage zur Cross-Flow Mikrofiltration
(C) der Anlage zur Diafiltration
(D) dem Radial-Typ Bioreaktor.
Die erfindungsgemäßen Verfahrensschritte a-d (gemäß Text) verteilen sich darauf wie folgt;
a) und b) werden im Fermenter (A), b) wird in Anlage (B), c) und d) werden im Radial-Typ Bioreaktor (D) durchgeführt. Die Anlage zur Diafiltration dient der Konditionierung des Enzymrohextraktes.
Weitere Einzelheiten der Anlage sind:

| | |
|---|---|
| (1) | Sterilfilter/Filterkerzen |
| (2) | Regel-, Absperrventile |
| (3) | Pumpe mit Angabe der Förderrichtung |
| (4) | Vorratsbehälter der Diafiltration. |
| (5),(6),(7), | Meßsonden am Fermenter, z.B. Druck, Temperatur, Probenahme Gas, Flüssig, |
| (8) | Probesonde am Vorratsgefäß |
| (9),(10) | Thermostatzulauf- und -ablauf am Radial-Typ Bioreaktor |
| (11) | Rührwerk mit Sterilbelüftung am Fermenter. |

Weitere Verfahrensschritte an der Reaktoranlage sind:

| | |
|---|---|
| e) | Zugabe von Nährlösung über kontinuierliche Sterilisation, Impfgut |
| f) | Zugabe von Salzlösung, z.B. NaCl, KCl |
| g) | Zugabe von oberflächenaktiven Reagentien |
| h) | Zugabe von Konditionierlösung, z.B. 1 mM Kaliumphosphat, pH 7,0 |
| i) | Durchsatz von Saccharoselösung durch den Radial-Typ Bioreaktor und |
| k) | Abführung des Produktstroms zur Kristallisation der Isomaltulose |

8

1)   Auslauf des Saccharose-Mutase-freien Enzymrohextrakts nach Beladung des Radial-Typ Bioreaktors.

m)   Sterilisation der Teilanlage durch Dampf.


Fig. 2a und b

Querschnitt durch den Radial-Typ Bioreaktor.

Die mit dem Enzym selektiv beladene, membranähnliche Träger- oder Gewebematrix wird entweder zylindrisch aufgewickelt (2a) oder in Form konzentrisch angeordneter Zylinderfolien (2b) verwendet. Die gewickelte Form (2a) ist eine leichter zu fertigende Näherung der konzentrischen Idealform. In beiden Fällen führt der Massenstrom jedoch durch die Gewebeschichten von außen radial nach innen zur Zylinderachse hin. Die gewickelte Form ist besonders mit zunehmender Durchflußrate eine gute Näherung der konzentrischen Form.


Fig. 3

Saccharose-Mutase Radial-Typ Bioreaktor:

Durchsatz und Umsetzung von Saccharose und Stabilität des trägergebundenen Enzyms (vergleiche Beispiel 1).

Ordinaten:

$\Delta$ - $\Delta$   : Rest-Saccharose in %

o - o   : Durchflußgeschwindigkeit in ml/h

o - o   : Isomaltulose in kg/Tag.

Auf der Abszisse ist die Zeit in Tagen angegeben.


**Ansprüche**

1.   Kontinuierliches Verfahren zur enzymatischen Herstellung von Isomaltulose, dadurch gekennzeichnet, daß man in einem steril führbaren Bioreaktorsystem

a) durch Fermentation Saccharose-Mutase-bildender Mikroorganismen Saccharose-Mutase herstellt, wobei die Zellen bereits vor Ende der logarithmischen Wachstumsphase mit Salzlösung und oberflächenaktiven Reagentien behandelt werden,

b) in an sich bekannter Weise durch Zellaufschluß und Cross-Flow-Mikrofiltration einen zellfreien Enzymrohextrakt herstellt,

c) die Saccharose-Mutase aus diesem Rohextrakt durch selektive Bindung an eine anionisierbare Trägermatrix gleichzeitig reinigt und immobilisiert, und

d) die dabei erhaltene Matrix mit Saccharose in Berührung bringt.


2.   Verfahren gemäß Anspruch 1, dadurch gekennzeichnet, daß für den Verfahrensschritt c) eine sulfonsaure Kationenaustauscher-Matrix verwendet wird.


3.   Verfahren gemäß Ansprüchen 1 und 2, dadurch gekennzeichnet, daß der Verfahrensschritt c) im pH-Bereich 4-12 und bei Salzkonzentrationen kleiner als 0,2 M, bevorzugt 1 $\mu$M bis 10 mM, erfolgt.


4.   Verfahren Gemäß Ansprüchen 1 bis 3, dadurch gekennzeichnet, daß der Verfahrensschritt b) durch Behandlung der Fermentationslösung im pH-Bereich 4-12 und bei Salzkonzentrationen von 0,1 M bis 3,0 M, bevorzugt in Gegenwart von Alkalichlorid, erfolgt.


5.   Verfahren gemäß Anspruch 4, dadurch gekennzeichnet, daß der Verfahrensschritt b) in Gegenwart oberflächenaktiver Reagentien im Konzentrationsbereich 0,1-5,0 % (w/v) erfolgt.


6.   Verfahren gemäß Anspruch 2, dadurch gekennzeichnet, daß als sulfonsaure Kationenaustauscher-Matrix ein Cellulosegewebe in Patronen- bzw. Kartuschenform verwendet wird, welches durch bifunktionelle Polymere vernetzt ist und über Vinylpolymere gebundene funktionelle sulfonsaure Gruppen aufweist, wobei das Trägermaterial zur Zylinderform mit oder ohne Abstandhalter zwischen den Cellulosegewebeschichten aufgewickelt ist und radial von außen nach innen durchströmbar vorliegt.


7.   Verfahren gemäß Anspruch 1, dadurch gekennzeichnet, daß im Verfahrensschritt a) das Enzym Saccharose-Mutase in an sich bekannter Weise durch Fermentation von Protaminobacter rubrum produziert wird.

8. Verfahren gemäß Anspruch 1, dadurch gekennzeichnet, daß im Verfahrensschritt a) das Enzym Saccharose-Mutase in an sich bekannter Weise durch Fermentation von Serratia plymuthica produziert wird.

9. Verfahren gemäß Anspruch 1, dadurch gekennzeichnet, daß im Verfahrensschritt a) das Enzym Saccharose-Mutase in an sich bekannter Weise durch Fermentation eines Mikroorganismus der Gattung Erwinia produziert wird.

10. Verfahren gemäß Anspruch 1, dadurch gekennzeichnet, daß der Verfahrensschritt d) in an sich bekannter Weise im pH-Bereich 4-8, bevorzugt 4,5-7,5, mit einer Saccharoselösung der Konzentration 10 - 70 %, bevorzugt 30 - 60 %, und im Temperaturbereich 10 - 40°C, bevorzugt 25 - 35°C, erfolgt.

**Claims**

1. A continuous process for enzymatic production of isomaltulose, characterised in that, in a bioreactorsystem that can be operated under sterile conditions,
   a) saccharose mutase is formed by fermentation of saccharose mutase forming microorganisms, the cells being treated with a salt solution and surface-active reagents before the end of the logarithmic growth phase,
   b) a cell-free crude enzyme extract is produced in known manner by breaking up the cells and cross-flow microfiltration,
   c) the saccharose mutase from this crude extract is simultaneously purified and immobilised by selective binding to an anionisable carrier matrix, and
   d) the matrix thus obtained is brought into contact with saccharose.

2. A process according to claim 1, characterised in that a sulphonic acid cation-exchange matrix is used for step c).

3. A process according to claim 1 and claim 2, characterised in that step c) takes place in the pH range 4 - 12 and at salt concentrations less than 0.2 M, preferably 1 $\mu$M to 10 mM.

4. A process according to any one of claims 1 to 3, characterised in that step b) takes place by treatment of the fermentation solution in the pH range 4 - 12 and at salt concentrations of 0.1 M to 3.0 M, preferably in the presence of alkali chloride.

5. A process according to claim 4, characterised in that step b) takes place in the presence of surface-active reagents in the concentration range 0.1 - 5.0% (w/v).

6. A process according to claim 2, characterised in that the sulphonic acid cation-exchange matrix used is a cellulose fabric, in the form of a cartridge or cartouche, which is cross-linked by means of bifunctional polymers and comprises sulphonic acid functional groups combined by means of vinyl polymers, the carrier material being wound into cylindrical shape with or without spacers between the layers of cellulose fabric and being permeable to radial flow from the outside inwards.

7. A process according to claim 1, characterised in that in step a) the enzyme saccharose mutase is produced in known manner by fermentation of Protaminobacter rubrum.

8. A process according to claim 1, characterised in that in step a) the enzyme saccharose mutase is produced in known manner by fermentation of Serratia plymuthica.

9. A process according to claim 1, characterised in that in step a) the enzyme saccharose mutase is produced in known manner by fermentation of a microorganism of the genus Erwinia.

10. A process according to claim 1, characterised in that step a) takes place in known manner in the pH range 4 - 8, preferably 4.5 - 7.5, using a saccharose solution concentration 10 - 70%, preferably 30 - 60%, and in the temperature range 10 - 40° C, preferably 25 - 3° C.

## Revendications

1. Procédé continu de préparation enzymatique d'isomaltulose, caractérisé par le fait que, dans un système de bioréacteur que l'on peut faire fonctionner de façon stérile
    a) on prépare par fermentation de micro-organismes générateurs de la mutase de saccharose, les cellules étant traitées au moyen d'une solution saline et de réactifs tensio-actifs avant la fin de la phase de croissance logarithmique,
    b) on prépare de manière connue par désagrégation des cellules et par microfiltration à courant transversal un extrait enzymatique exempt de cellules,
    c) la mutase de saccharose est simultanément purifiée et fixée partir de cet extrait brut par fixation sélective sur une matrice de support susceptible d'être dotée de propriétés anioniques, et
    d) la matrice ainsi obtenue est mise en contact avec du saccharose.

2. Procédé selon la revendication 1, caractérisé par le fait que l'on utilise pour la phase c) du procédé une matrice sulfonique échangeuse de cations.

3. Procédé selon les revendications 1 et 2, caractérisé par le fait que la phase c) du procédé s'effectue dans le domaine de pH allant de à 12 et avec des concentrations salines inférieures à 0,2 M, de préférence 1 $\mu$M à 10 mM.

4. Procédé selon les revendications 1 à 3, caractérisé par le fait que la phase b) du procédé s'effectue par traitement de la solution de fermentation dans le domaine de pH de 4 à 12 et avec des concentrations salines de 0,1 M à 3,0 M, de préférence en présence de chlorure alcalin.

5. Procédé selon la revendication 4, caractérisé par le fait que la phase b) du procédé est effectuée en présence de réactifs tensio-actifs avec le domaine de concentration de 0,1 à 5,0 % (w/v).

6. Procédé selon la revendication 2, caractérisé par le fait que l'on emploie en tant que matrice sulfonique échangeuse de cations un tissu de cellulose sous forme de cartouches qui est réticulé au moyen de polymères bifonctionnels et qui présente des groupes fonctionnels sulfoniques reliés au moyen de polymères vinyliques, le matériau de support étant enroulé pour obtenir une forme cylindrique avec ou sans organe de maintien d'écart entre les couches en tissu de cellulose et se présentant comme étant susceptible d'être traversé radialement de l'extérieur vers l'intérieur.

7. Procédé selon la revendication 1, caractérisé par le fait que dans la phase a) du procédé l'enzyme mutase de saccharose est produit de façon connue en elle-même par fermentation de protaminobacter rubrum.

8. Procédé selon la revendication 1, caractérisé par le fait que dans la phase a) du procédé l'enzyme mutase de saccharose est produit de façon connue en elle-même par fermentation de serratia plymuthica.

9. Procédé selon la revendication 1, caractérisé par le fait que dans la phase a) du procédé l'enzyme mutase de saccharose est produit de façon connue en elle-même par fermentation d'un microorganisme appartenant au genre Erwinia.

10. Procédé selon la revendication 1, caractérisé par le fait que la phase d) du procédé est réalisée de façon connue en ellemême dans un domaine de pH allant de 4 à 8, de préférence de 4,5 à 7,5 %, avec une solution de saccharose à la concentration de 10 à 70 %, de préférence 30 à 60 % et dans un domaine de températures de 10 à 40° C, de préférence 25 à 35° C.

Fermentation

Cross-Flow
Mikrofiltration

Zellaufschluss
Cross-Flow
Mikrofiltration
Diafiltration

Zellkonzentrat

Zellaufschluss
Cross-Flow
Mikrofiltration
Diafiltration

Konditionierter
Enzymrohextrakt

anionisierbare
Trägermatrix
z.B. AMF-ZetaPrep-SP

Saccharose ⟶ Immobilisierte
Saccharose Mutase
Radial-Typ Bioreaktor ⟶ Isomaltulose

Saccharose Mutase freier
Enzymrohextrakt
Abwasser

FIG. 1a

FIG.1b

EP 0 200 069 B1

FIG. 2a

FIG. 2b

FIG. 3